# FASCICULE DE BREVET EUROPEEN

(11) **EP 0 687 663 B1**
(45) Date de publication et mention de la délivrance du brevet: **25.08.1999**
(21) Numéro de dépôt: 95420155.4
(22) Date de dépôt: 12.06.1995
(51) Int. Cl.: C07C 55/02, C07C 55/10, C07C 55/12, C07C 51/487

(54) **Procédé de séparation de diacides aliphatiques à partir de leurs mélanges avec l'acide adipique**
Verfahren zur Trennung von aliphatischen Dicarbonsäuren aus ihren Gemischen mit Adipinsäure
Process for separating aliphatic dicarboxylic acids from their mixtures with adipic acid

(30) Priorité: 14.06.1994 FR 9407505
(43) Date de publication de la demande: 20.12.1995
(73) Titulaire: RHODIA FIBER & RESIN INTERMEDIATES, 92408 Courbevoie Cédex (FR)
(72) Inventeur: Denis, Philippe, F-69150 Decines (FR); Patois, Carl, F-69003 Lyon (FR); Perron, Robert, F-69390 Charly (FR)
(74) Mandataire: Vignally, Noel

(56) Documents cités:
- DE-A- 1 938 103
- FR-A- 1 316 914

## Description

La présente invention concerne un procédé de séparation d'un ou plusieurs diacides aliphatiques, à partir de mélanges contenant de l'acide adipique.

L'acide adipique est l'une des deux matières premières à la base des polyamides-66.

Il existe différents procédés de préparation de l'acide adipique, dont certains sont industriels et d'autres au stade des recherches ou du développement.

Un procédé qui fait l'objet de différentes demandes de brevets, consiste à hydroxycarbonyler le butadiène pour former les acides penténoïques, puis à hydroxycarbonyler ces derniers pour former l'acide adipique. Lors de la deuxième réaction d'hydroxycarbonylation, il se forme également des quantités, variables selon les conditions de mise en oeuvre du procédé, d'acide méthyl-glutarique, d'acide éthyl-succinique et d'acide diméthyl-succinique.

Ces divers diacides aliphatiques saturés ramifiés peuvent être plus ou moins valorisables, mais il importe dans tous les cas de les séparer le mieux possible de l'acide adipique, qui demeure le composé le plus important, qualitativement et quantitativement.

On peut utiliser des procédés de fractionnement des mélanges, par exemple par cristallisation, pour séparer les diacides ramifiés de l'acide adipique. Cependant, s'agissant d'isomères de l'acide adipique, cette technique ne permet pas une séparation aisée convenable, en raison des propriétés physiques très proches de tous ces diacides.

Le brevet DE-A-1938103 décrit un procédé de séparation d'un mélange de diacides carboxyliques provenant de l'oxydation nitrique du cyclohexanol et/ou de la cyclohexanone consistant à cristalliser la majeure partie de l'acide adipique, à évaporer la solution d'acide nitrique, à vaporiser le résidu, à déshydrater l'acide succinique en anhydride succinique et l'acide glutarique en anhydride glutarique et à distiller les anhydrides formés.

Le brevet FR-A-1316914 décrit un procédé de traitement d'un mélange de diacides organiques comprenant l'acide succinique, l'acide glutarique et l'acide adipique, ledit procédé consistant à transformer l'acide succinique et l'acide glutarique en leurs anhydrides respectifs par chauffage sous pression réduite et distillation de l'eau formée et des anhydrides. Préalablement à ce traitement, l'acide nitrique et l'eau présents dans la solution initiale sont évaporés.

La présente invention concerne un procédé de séparation de diacides aliphatiques saturés, ramifiés, comportant 6 atomes de carbone, à partir de mélanges contenant également au moins de l'acide adipique, grâce à la transformation d'au moins une partie desdits diacides en anhydrides correspondants.

Plus précisément, elle consiste en un procédé de séparation d'au moins une partie d'un ou plusieurs diacides aliphatiques saturés ramifiés ayant 6 atomes de carbone, à partir de mélanges les contenant avec au moins de l'acide adipique, caractérisé en ce que ledit ou lesdits diacides sont transformés au moins partiellement en anhydrides correspondants.

Dans le présent texte, les mélanges de diacides auxquels s'applique l'invention englobent aussi bien les mélanges d'un seul de ces diacides avec l'acide adipique que les mélanges de plusieurs d'entre-eux avec l'acide adipique et peuvent bien entendu contenir d'autres composés. Par commodité, le terme "diacides" sera généralement utilisé, tout en s'appliquant éventuellement à un seul diacide. De même ce terme diacides n'englobera pas, sauf mention contraire, l'acide adipique. Enfin l'opération de transformation des diacides en anhydrides correspondants sera également appelée "anhydridisation".

Les diacides aliphatiques saturés mis en oeuvre dans le procédé de l'invention sont l'acide méthyl-2 glutarique et/ou l'acide éthyl-2 succinique et/ou l'acide diméthyl-succinique d'autre part.

L'anhydridisation des diacides aliphatiques saturés peut être réalisée par chauffage du mélange les contenant, à une température égale ou supérieure à 60 °C.

Pour faciliter la déshydratation des diacides, il est en général préférable de séparer, notamment par distillation, les anhydrides formés ou l'eau formée, au cours de l'anhydridisation ou après celle-ci. Mais cela n'est pas indispensable et les anhydrides formés peuvent aussi demeurer dans le mélange réactionnel et n'être séparés de l'acide adipique et des autres composés présents, par un moyen connu tel que par exemple une distillation ou une cristallisation, que lors d'une opération ultérieure.

Le chauffage et l'éventuelle distillation pouvant être effectués sous une pression égale à la pression atmosphérique, supérieure ou inférieure à celle-ci, la température à laquelle sera conduite l'anhydridisation pourra varier en fonction de cette pression, ainsi que de la composition du mélange à traiter.

Cependant à titre indicatif, le procédé sera généralement mis en oeuvre à une température de 80 °C à 350 °C.

Le procédé peut avantageusement être conduit en présence d'un catalyseur acide homogène, c'est-à-dire soluble dans le milieu réactionnel, ayant un pKa inférieur ou égal à 5, tel que par exemple l'acide sulfurique, l'acide iodhydrique, l'acide paratoluènesulfonique, l'acide trifluoroacétique, l'acide trifluorométhanesulfonique, ou hétérogène tel que par exemple le phosphate de bore, la zircone ou les résines sulfonées comme celles commercialisées sous la marque Nafion ou encore les argiles acides comme notamment les smectites telles que par exemple les montmorillonites, les beidellites, les nontronites, les hectorites, les stévensites et les saponites.

La quantité de catalyseur acide homogène ou hétérogène peut varier très largement puisque la présence d'un tel acide n'est pas indispensable. Elle peut donc être telle que l'on ait un rapport molaire acide/diacides à anhydridiser de 0 à 10. De préférence le catalyseur acide homogène ou hétérogène sera en quantité telle que le rapport molaire acide/diacides à anhydridiser soit de 0,0005 à 1. Lorsque l'on met en oeuvre une résine acide sulfonée le rapport molaire sera considéré entre les fonctions acide sulfonique de ladite résine et les diacides à anhydridiser. Lorsque l'on utilise une argile acide, un rapport pondéral argile acide/diacides à anhydridiser d'au moins 5 % peut être mis en oeuvre, mais il est bien évident que ce rapport peut être très variable selon la réalisation du procédé. En particulier en cas de mise en oeuvre en mode continu, la notion de rapport pondéral n'a plus de signification et est remplacée par celle de temps de contact.

Une autre variante intéressante de l'invention consiste à opérer en présence dans le milieu réactionnel d'un anhydride ou de plusieurs anhydrides, comme les anhydrides des acides aliphatiques monocarboxyliques ou polycarboxyliques, le point d'ébullition de ces acides devant être inférieur à celui de l'acide adipique (c'est à dire environ 265 °C sous 13,3 KPa) afin que cesdits acides formés à partir de leurs anhydrides puissent être séparés par distillation. Les anhydrides utilisés sont notamment les anhydrides des acides monocarboxyliques ou polycarboxyliques, ayant de 2 à 8 atomes de carbone, tels que par exemple les anhydrides des acides acétique, propionique, succinique, valérique, éthyl-2 succinique, diméthyl-succinique, méthyl-butanoïques, méthyl-buténoïques ou penténoïques ainsi que les éventuels anhydrides mixtes de ces acides.

Il est évident que le choix des anhydrides éthyl-2 succinique et diméthyl-succinique ne se justifie que si les diacides à séparer de l'acide adipique ne sont pas respectivement les acides éthyl-2 succinique et diméthyl-succinique.

Comme pour la variante précédente, la quantité d'anhydride éventuellement rajoutée dans le milieu réactionnel, pour faciliter l'anhydridisation des diacides, peut varier dans de très larges limites puisque la présence d'un tel composé n'est pas indispensable, même si cela constitue une variante préférentielle de l'invention. La quantité d'anhydride peut donc être telle que l'on ait un rapport molaire anhydride/diacides à anhydridiser de 0 à 10. De préférence le rapport molaire anhydride/diacides à anhydriser est de 0,5 à 2 et de manière encore plus préférentielle de 1 à 1,5.

Comme indiqué précédemment, les mélanges de diacides mis en oeuvre dans le procédé de l'invention peuvent être des mélanges provenant d'une origine quelconque; ils peuvent notamment provenir de la récupération des diacides dans des eaux de recristallisation de l'acide adipique ou encore des résidus de distillation des diacides ou être constitués de fractions plus ou moins pures issues d'opérations de cristallisation, de raffinage ou de distillation réalisées dans le cadre de la préparation d'acide adipique.

Ils peuvent également être directement issus des procédés de synthèse de l'acide adipique. C'est notamment le cas des mélanges d'acide adipique avec l'acide méthyl-2 glutarique et/ou l'acide éthyl-2 succinique et/ou l'acide diméthyl-succinique, obtenus lors de l'hydroxycarbonylation des acides penténoïques ou le cas échéant déjà lors de l'hydroxycarbonylation du butadiène ou de ses dérivés. Ces mélanges pourront contenir également et selon l'évolution des phases du procédé de synthèse, des quantités plus ou moins importantes du catalyseur utilisé, tel que par exemple l'iridium ou des composés de l'iridium, le rhodium ou des composés du rhodium, le palladium ou des composés du palladium, cette dernière famille de catalyseurs étant plus particulièrement utilisée pour l'hydroxycarbonylation du butadiène ou de ses dérivés. lls pourront aussi, renfermer des composés mis en oeuvre tels que les acides penténoïques, les solvants éventuels, les cocatalyseurs, ainsi que des composés formés lors de la synthèse de l'acide adipique tels que notamment l'acide valérique, les acides méthyl-butanoïques, les acides méthyl-buténoïques, la gamma-valérolactone.

La présence de catalyseur, notamment l'iridium ou les composés de l'iridium, le rhodium ou les composés du rhodium, le palladium ou les composés du palladium, ainsi que des cocatalyseurs ou promoteurs respectifs de ces catalyseurs, peut jouer un rôle bénéfique dans l'anhydridisation des diacides. Qu'ils proviennent des mélanges réactionnels issus des procédés d'hydroxycarbonylation des acides penténoïques ou du butadiène ou qu'ils soient éventuellement rajoutés avant la mise en oeuvre du procédé d'anhydridisation, le catalyseur représente en moles de métal par rapport aux moles de diacides à traiter de 0 % à 20 %, de préférence de 0 % à 10 % et encore plus préférentiellement 0 % à 5 %.

Les quantités des différents composés indiqués précédemment dans les mélanges réactionnels issus de l'hydroxycarbonylation des acides penténoïques ou du butadiène ou de ses dérivés ne sont pas critiques. On peut trouver des illustrations des quantités que l'on peut obtenir, dans les différents brevets décrivant ces procédés de synthèse de l'acide adipique, tels que de manière non limitative les brevets EP-A-0 477 112, EP-A-0 478 472, EP-A-0 493 273, EP-A-0 511 126, EP-A-0 536 064.

On peut également dans le cadre du procédé d'anhydridisation de l'invention, rajouter au mélange de diacides à traiter un solvant, qui peut être l'un des composés indiqués précédemment ou être différent des composés présents dans le mélange réactionnel issu du procédé de synthèse de l'acide adipique et des diacides à anhydridiser.

Ces solvants peuvent être choisis notamment parmi les hydrocarbures aliphatiques ou cycloaliphatiques, les hydrocarbures aliphatiques ou cycloaliphatiques halogénés, en particulier chlorés, les hydrocarbures aromatiques, les hydrocarbures aromatiques halogénés, en particulier chlorés, les éthers aliphatiques ou aromatiques ou mixtes, les acides carboxyliques en particulier aliphatiques, les acides carboxyliques halogénés.

A titre d'exemples non limitatifs de tels solvants, on peut citer le benzène, le toluène, les xylènes, le n-hexane, le dichlorométhane, le dichloro-1,2-éthane, le cyclohexane, le chlorobenzène, le diphényléther, le dibutyléther, les acides penténoïques, l'acide valérique, la gamma-valérolactone, l'acide acétique, l'acide propionique, l'acide trifluoroacétique.

Ces solvants sont rajoutés dans le but de diluer les diacides et favoriser leur anhydridisation. lls peuvent aussi être choisis pour permettre la distillation azéotropique de l'eau formée lors de l'anhydridisation.

La quantité de solvant peut représenter de 0% à 95% du poids total du mélange contenant les diacides.

Généralement les diacides à anhydriser représentent de 10% à 90% en poids par rapport au poids total diacides/acide adipique dans le mélange à traiter.

La transformation des diacides en leurs anhydrides permet de pouvoir les séparer plus aisément de l'acide adipique, notamment par distillation en raison des points d'ébullition beaucoup plus bas des anhydrides par rapport à leurs diacides respectifs.

Les exemples qui suivent illustrent l'invention.

### EXEMPLE 1

Dans un ballon en verre de 50 ml, on charge un mélange contenant 50 millimoles (mmol) d'acide méthyl-2 glutarique, 25 mmol d'acide adipique et 50 mmol d'acide penténoïque (servant de solvant) auquel on additionne 2 mmol d'acide sulfurique concentré.

Le mélange est agité, chauffé à 130°C et maintenu à cette température pendant 30 minutes.

Par distillation sous pression réduite (environ 1000 Pa), on obtient 80 % d'anhydride correspondant par rapport à l'acide méthyl-2 glutarique chargé.

### EXEMPLE 2

L'exemple 1 est répété dans les mêmes conditions opératoires, mais l'acide méthyl-2 glutarique est remplacé par la même quantité molaire d'acide éthyl-2 succinique. Par distillation on obtient 100 % d'anhydride correspondant par rapport à l'acide éthyl-2 succinique chargé.

### EXEMPLE 3

Dans un ballon en verre de 50 ml, on charge un mélange contenant 34 mmol d'acide méthyl-2 glutarique, 39 mmol d'anhydride de l'acide éthyl-2 succinique et 25 mmol d'acide adipique.

Le mélange est agité, chauffé à 120°C et maintenu à cette température pendant une heure. Par distillation comme dans l'exemple 1, on obtient 2 mmol d'anhydride correspondant à l'acide méthyl-2 glutarique.

### EXEMPLE 4

L'exemple 1 est répété dans les mêmes conditions opératoires, mais l'acide sulfurique est remplacé par 2,5 mmol de phosphate de bore. Par distillation comme dans l'exemple 1, on obtient 9 mmol d'anhydride correspondant à l'acide méthyl-2 glutarique.

### EXEMPLE 5

Dans un autoclave de 125 ml, on charge successivement :
- 0,8 mmol de RhCl(cod){cod=1,5-cyclooctadiène}
- 8 mmol de Hl (à 57% dans l'eau)
- 45 mmol d'eau (apportés par la solution de Hl)
- 20 mmol d'acide adipique
- 39 mmol d'acide éthyl-2 succinique
- 45 ml d'acide acétique.

Le mélange est porté sous agitation pendant 5 h à 230 °C sous 50 bar de CO (pression totale en température).

Par distillation comme dans l'exemple 1, on obtient 3 mmol d'anhydride correspondant à l'acide éthyl-2 succinique.

### EXEMPLE 6

Dans un autoclave de 125 ml, on charge successivement :
- 0,8 mmol de IrCl(cod)₂
- 1,6 mmol de Hl (à 57% dans l'eau)
- 9 mmol d'eau (apportés par la solution de Hl)
- 20 mmol d'acide adipique
- 39 mmol d'acide méthyl-2 glutarique
- 45 ml d'acide acétique.

Le mélange est porté sous agitation pendant 5 h à 230 °C sous 50 bar de CO (pression totale en température).

Par distillation comme dans l'exemple 1, on obtient 3 mmol d'anhydride correspondant à l'acide éthyl-2 succinique (via une isomérisation de l'acide méthyl-2 glutarique en acide éthyl-2 succinique).

### EXEMPLE 7

Dans un ballon en verre de 50 ml, on charge successivement 50 mmol d'acide méthyl-2 glutarique et 11,6 g d'une solution provenant d'un essai d'hydroxycarbonylation de l'acide pentène-3-oïque et contenant :
- 0,0386 mmol de Ir sous forme soluble
- 0,0907 mmol de Hl
- 3,6 mmol de gamma-valérolactone
- 67 mmol d'acide penténoïque
- 23,6 mmol d'acide adipique
- 6 mmol d'acide méthyl-2 glutarique
- 1,8 mmol d'acide éthyl-2 succinique.

Le mélange est porté sous agitation pendant 30 min à 130 °C, puis 2 h à 200 °C.

Par distillation comme dans l'exemple 1, on obtient 4,5 mmol d'anhydride correspondant à l'acide méthyl-2 glutarique et 1,8 mmol d'anhydride correspondant à l'acide éthyl-2 succinique.

### EXEMPLE 8

Dans un ballon en verre de 50 ml, on charge 10 g d'une solution contenant :
- 0,016 mmol de lr sous forme soluble
- 28 mmol d'acide penténoïque
- 8,2 mmol d'acide adipique
- 37,7 mmol d'acide méthyl-2 glutarique
- 3,4 mmol d'acide éthyl-2 succinique.

Le mélange est chauffé pendant 90 min à 220 °C, sous une pression réduite (430 Pa).

On constate par dosage que 23 % de l'acide méthyl-2 glutarique et 100 % de l'acide éthyl-2 succinique mis en oeuvre ont été transformés en leurs anhydrides respectifs, dont une partie importante a été distillée au cours de la réaction d'anhydridisation.

### EXEMPLE 9

Dans un ballon en verre de 50 ml, on charge 10 g d'une solution contenant :
- 0,026 mmol de Ir sous forme soluble
- 41 mmol d'acide penténoïque
- 17,1 mmol d'acide adipique
- 19,2 mmol d'acide méthyl-2 glutarique
- 4,1 mmol d'acide éthyl-2 succinique
- 24 mmol d'anhydride acétique.

Le mélange est chauffé pendant 60 min à reflux (environ 120 °C), puis on distille sous 1300 Pa, jusqu'à une température de 220 °C dans le mélange réactionnel.

On constate par dosage que 99 % de l'acide méthyl-2 glutarique et 99 % de l'acide éthyl-2 succinique mis en oeuvre ont été transformés et distillés.

### EXEMPLE 10

Dans un ballon en verre de 500 ml, on charge :
- 200 mmol d'acide adipique
- 200 mmol d'acide méthyl-2 glutarique
- 250 ml de méta-xylène
- 10 mmol d'acide sulfurique.

Le mélange est chauffé sous agitation pendant 4 h à reflux du xylène et l'eau formée est distillée dans une colonne azéotropique (100 % de la théorie).

La distillation effectuée sur le mélange déshydraté a permis de séparer l'anhydride de l'acide méthyl-2 glutarique formé (90 % en moles par rapport à l'acide méthyl-2 glutarique chargé).

### ESSAI COMPARATIF 1

Dans un ballon en verre de 50 ml, on charge un mélange contenant 15 g (103 mmol) d'acide méthyl-2 glutarique, 2 g (13,7 mmol) d'acide éthyl-2 succinique et 10 g (68,5 mmol) d'acide adipique.

Le mélange est agité, chauffé à 120°C et maintenu à cette température pendant 2 heures. Puis on le maintient pendant 6 heures à 160 °C sous pression réduite (1400 Pa).

On constate par dosage du distillat obtenu que 2,2 % de l'acide méthyl-2 glutarique et 93 % de l'acide éthyl-2 succinique mis en oeuvre ont été distillés sous forme de leurs anhydrides correspondants respectifs.

### EXEMPLE 11

Dans un ballon en verre de 50 ml, on charge un mélange contenant 15 g (103 mmol) d'acide méthyl-2 glutarique, 2 g (13,7 mmol) d'acide éthyl-2 succinique et 10 g (68,5 mmol) d'acide adipique, ainsi que 3 g de résine sulfonée de marque Nafion NR 50 (10-35 mesh).

Le mélange est agité, chauffé à 120°C et maintenu à cette température pendant 2 heures. Puis on le maintient pendant 6 heures à 160 °C sous pression réduite (1400 Pa).

On constate par dosage du distillat obtenu que 4 % de l'acide méthyl-2 glutarique et 99 % de l'acide éthyl-2 succinique mis en oeuvre ont été distillés sous forme de leurs anhydrides correspondants respectifs.

### EXEMPLE 12

Dans un ballon en verre de 50 ml, on charge un mélange contenant 15 g (103 mmol) d'acide méthyl-2 glutarique, 2 g (13,7 mmol) d'acide éthyl-2 succinique et 10 g (68,5 mmol) d'acide adipique, ainsi que 3 g d'argile acide montmorillonite KSF.

Le mélange est agité, chauffé à 120°C et maintenu à cette température pendant 2 heures. Puis on le maintient pendant 6 heures à 160 °C sous pression réduite (1400 Pa).

On constate par dosage du distillat obtenu que 24 % de l'acide méthyl-2 glutarique et 92 % de l'acide éthyl-2 succinique mis en oeuvre ont été distillés sous forme de leurs anhydrides correspondants respectifs.

### EXEMPLE 13

Dans un autoclave de 125 ml, on charge successivement :
- 0,9 mmol de PdCl₂
- 7 mmol de chlorobutène
- 92,5 mmol d'eau
- 92,5 mmol de butadiène
- 10 mmol d'acide pentène-3-ïque
- 34 mmol d'acide adipique
- 68,5 mmol d'acide méthyl-2 glutarique.

Le mélange est porté sous agitation pendant 5 h 30 à 100 °C sous 200 bar de CO (pression totale en température).

Par distillation comme dans l'exemple 1, on obtient 13 mmol d'anhydride correspondant à l'acide méthyl-2 glutarique ainsi que 1 mmol d'anhydride correspondant à l'acide éthyl-2 succinique.

## Revendications

1. Procédé de séparation d'au moins une partie d'un ou plusieurs diacides aliphatiques saturés ramifiés ayant 6 atomes de carbone, à partir de mélanges les contenant avec au moins de l'acide adipique, caractérisé en ce que ledit ou lesdits diacides sont transformés au moins partiellement en anhydrides correspondants,
- en présence d'un catalyseur acide homogène, c'est-à-dire soluble dans le milieu réactionnel, ayant un pKa inférieur ou égal à 5 ou d'un catalyseur acide hétérogène,
- ou en présence dans le milieu réactionnel d'un anhydride ou de plusieurs anhydrides, comme les anhydrides des acides aliphatiques monocarboxyliques ou polycarboxyliques, le point d'ébullition de ces acides étant inférieur à celui de l'acide adipique,
- ou d'un catalyseur choisi parmi l'iridium, les composés de l'iridium, le rhodium, les composés du rhodium, le palladium, les composés du palladium.

2. Procédé selon la revendication 1, caractérisé en ce que les diacides aliphatiques saturés mis en oeuvre sont l'acide méthyl-2 glutarique et/ou l'acide éthylsuccinique et/ou l'acide diméthyl-succinique.

3. Procédé selon l'une des revendications 1 ou 2, caractérisé en ce que l'anhydridisation des diacides aliphatiques saturés est réalisée par chauffage du mélange les contenant, à une température égale ou supérieure à 60 °C.

4. Procédé selon l'une des revendications 1 à 3, caractérisé en ce qu'il comprend la distillation des anhydrides formés ou de l'eau formée, soit au cours de l'anhydridisation, soit après celle-ci, ou la séparation des anhydrides par un autre moyen tel que la cristallisation.

5. Procédé selon l'une des revendications 1 à 4, caractérisé en ce qu'il est mis en oeuvre à une température de 80 °C à 350 °C.

6. Procédé selon l'une des revendications 1 à 5, caractérisé en ce qu'il est conduit en présence d'un catalyseur acide, homogène tel que l'acide sulfurique, l'acide iodhydrique, l'acide para-toluènesulfonique, l'acide trifluoroacétique, l'acide trifluorométhanesulfonique, ou hétérogène tel que le phosphate de bore, la zircone ou les résines sulfonées ou encore les argiles acides comme notamment les smectites telles que par exemple les montmorillonites, les beidellites, les nontronites, les hectorites, les stévensites et les saponites.

7. Procédé selon l'une des revendications 1 ou 6, caractérisé en ce que la quantité de catalyseur acide homogène ou hétérogène est telle que l'on a un rapport molaire acide/diacides à anhydridiser de 0,0005 à 10 et de préférence de 0,0005 à 1 ou un rapport pondéral argile acide/diacides à anhydridiser d'au moins 5 %.

8. Procédé selon l'une des revendications 1 à 5, caractérisé en ce qu'il est conduit en présence d'anhydride choisi parmi les anhydrides des acides monocarboxyliques ou polycarboxyliques, ayant de 2 à 8 atomes de carbone, tels que les anhydrides des acides acétique, propionique, succinique, valérique, éthyl-2 succinique, diméthyl-succinique, méthyl-butanoïques, méthyl-buténoïques ou penténoïques ainsi que les éventuels anhydrides mixtes de ces acides.

9. Procédé selon l'une des revendications 1 ou 8 caractérisé en ce que la quantité d'anhydride est telle que l'on a un rapport molaire anhydride/diacides à anhydridiser de 0,5 à 10, de préférence de 0,5 à 2 et de manière encore plus préférentielle de 1 à 1,5.

10. Procédé selon l'une des revendications 1 à 9, caractérisé en ce que les mélanges de diacides mis en oeuvre sont des mélanges provenant de la récupération des diacides dans des eaux de recristallisation de l'acide adipique ou encore des résidus de distillation des diacides ou être constitués de fractions plus ou moins pures issues d'opérations de cristallisation, de raffinage ou de distillation réalisées dans le cadre de la préparation d'acide adipique.

11. Procédé selon l'une des revendications 1 à 9, caractérisé en ce que les mélanges de diacides mis en oeuvre sont des mélanges issus des procédés de synthèse de l'acide adipique.

12. Procédé selon la revendication 11, caractérisé en ce que les mélanges mis en oeuvre sont des mélanges d'acide adipique avec l'acide méthyl-2 glutarique et/ou l'acide éthyl-2 succinique et/ou l'acide diméthyl-succinique, obtenus lors de l'hydroxycarbonylation des acides penténoïques ou de l'hydroxycarbonylation du butadiène ou de ses dérivés, et contenant éventuellement des quantités plus ou moins importantes du catalyseur utilisé, tel que l'iridium ou des composés de l'iridium, le rhodium ou des composés du rhodium, le palladium ou des composés du palladium et/ou des composés mis en oeuvre tels que les acides penténoïques, les solvants éventuels, les cocatalyseurs, ainsi que des composés formés lors de la synthèse de l'acide adipique tels que l'acide valérique, les acides méthyl-butanoïques, les acides méthyl-buténoïques,la gamma-valérolactone.

13. Procédé selon l'une des revendications 1 à 12, caractérisé en ce que l'on rajoute au mélange de diacides à traiter un solvant qui peut être l'un des composés présents dans le mélange réactionnel issu du procédé de synthèse de l'acide adipique et des diacides à anhydridiser ou être différent de ces composés.

14. Procédé selon la revendication 13, caractérisé en ce que le solvant mis en oeuvre est choisi parmi les hydrocarbures aliphatiques ou cycloaliphatiques, les hydrocarbures aliphatiques ou cycloaliphatiques halogénés, en particulier chlorés, les hydrocarbures aromatiques, les hydrocarbures aromatiques halogénés, en particulier chlorés, les éthers aliphatiques ou aromatiques ou mixtes, les acides carboxyliques en particulier aliphatiques, les acides carboxyliques halogénés.

15. Procédé selon l'une des revendications 13 ou 14, caractérisé en ce que le solvant est choisi parmi le benzène, le toluène, les xylènes, le n-hexane, le dichlorométhane, le dichloro-1,2-éthane, le cyclohexane, le chlorobenzène, le diphényléther, le dibutyléther, les acides penténoïques, l'acide valérique, la gammavalérolactone, l'acide acétique, l'acide propionique, l'acide trifluoroacétique.

## Claims

1. Process for the separation of at least some of one or more branched, saturated aliphatic diacids having 6 carbon atoms, from mixtures containing them with at least adipic acid, characterized in that the said diacid or diacids are at least partly converted into corresponding anhydrides,
- in the presence of a homogeneous acid catalyst, that is to say one which is soluble in the reaction medium, having a pKa below or equal to 5, or in the presence of a heterogeneous acid catalyst,
- or in the presence in the reaction medium of an anhydride or of several anhydrides, such as the anhydrides of monocarboxylic or polycarboxylic aliphatic acids, the boiling point of these acids being below that of adipic acid,
- or of a catalyst chosen from iridium, iridium compounds, rhodium, rhodium compounds, palladium and palladium compounds.

2. Process according to Claim 1, characterized in that the saturated aliphatic diacids used are 2-methylglutaric acid and/or ethylsuccinic acid and/or dimethylsuccinic acid.

3. Process according to either of Claims 1 and 2, characterized in that the anhydridization of the saturated aliphatic diacids is performed by heating the mixture containing them to a temperature equal to or above 60°C.

4. Process according to one of Claims 1 to 3, characterized in that it comprises the distillation of the anhydrides formed or of the water formed, either during the anhydridization, or after the latter, or the separation of the anhydrides by another means such as crystallization.

5. Process according to one of Claims 1 to 4, characterized in that it is carried out at a temperature of 80°C to 350°C.

6. Process according to one of Claims 1 to 5, characterized in that it is performed in the presence of a homogeneous acid catalyst such as sulphuric acid, hydriodic acid, para-toluenesulphonic acid, trifluoroacetic acid or trifluoromethanesulphonic acid, or a heterogeneous acid catalyst such as boron phosphate, zirconia or sulphonated resins, or alternatively acidic clays such as, in particular, smectites such as, for example, montmorillonites, beidellites, nontronites, hectorites, stevensites and saponites.

7. Process according to either of Claims 1 and 6, characterized in that the amount of homogeneous or heterogeneous acid catalyst is such that there is an acid/diacids to be anhydridized molar ratio of 0.0005 to 10 and preferably of 0.0005 to 1 or an acidic clay/diacids to be anhydridized weight ratio of at least 5 %.

8. Process according to one of Claims 1 to 5, characterized in that it is performed in the presence of anhydride chosen from anhydrides of monocarboxylic or polycarboxylic acids having from 2 to 8 carbon atoms, such as the anhydrides of acetic acid, propionic acid, succinic acid, valeric acid, 2-ethylsuccinic acid, dimethylsuccinic acid, methylbutanoic acids, methylbutenoic acids or pentenoic acids as well as the possible mixed anhydrides of these acids.

9. Process according to either of Claims 1 and 8, characterized in that the amount of anhydride is such that there is an anhydride/diacids to be anhydridized molar ratio of 0.5 to 10, preferably of 0.5 to 2 and even more preferably of 1 to 1.5.

10. Process according to one of Claims 1 to 9, characterized in that the diacid mixtures used are mixtures coming from the recovery of the diacids in recrystallization waters of adipic acid or alternatively from the diacid distillation residues, or they may consist of more or less pure fractions derived from crystallization, refining or distillation operations performed in the context of the preparation of adipic acid.

11. Process according to one of Claims 1 to 9, characterized in that the diacid mixtures used are mixtures derived from processes for the synthesis of adipic acid.

12. Process according to Claim 11, characterized in that the mixtures used are mixtures of adipic acid with 2-methylglutaric acid and/or 2-ethylsuccinic acid and/or dimethylsuccinic acid, which are obtained during the hydroxycarbonylation of the pentenoic acids or the hydroxycarbonylation of butadiene or derivatives thereof, and optionally containing larger or smaller amounts of the catalyst used, such as iridium or iridium compounds, rhodium or rhodium compounds, palladium or palladium compounds and/or compounds used such as pentenoic acids, possible solvents, and cocatalysts, as well as compounds formed during the synthesis of adipic acid, such as valeric acid, methylbutanoic acids, methylbutenoic acids and gamma-valerolactone.

13. Process according to one of Claims 1 to 12, characterized in that a solvent is added to the mixture of diacids to be treated, which solvent may be one of the compounds present in the reaction mixture derived from the process for the synthesis of adipic acid and the diacids to be anhydridized, or may be different from these compounds.

14. Process according to Claim 13, characterized in that the solvent used is chosen from aliphatic or cycloaliphatic hydrocarbons, halogenated, in particular chlorinated, aliphatic or cycloaliphatic hydrocarbons, aromatic hydrocarbons, halogenated, in particular chlorinated, aromatic hydrocarbons, aliphatic or aromatic or mixed ethers, carboxylic acids, in particular aliphatic carboxylic acids, and halogenated carboxylic acids.

15. Process according to either of Claims 13 and 14, characterized in that the solvent is chosen from benzene, toluene, xylenes, n-hexane, dichloromethane, 1,2-dichloroethane, cyclohexane, chlorobenzene, diphenyl ether, dibutyl ether, pentenoic acids, valeric acid, gamma-valerolactone, acetic acid, propionic acid and trifluoroacetic acid.

## Patentansprüche

1. Verfahren zur Abtrennung mindestens eines Teils einer oder mehrerer gesättigter aliphatischer verzweigter Dicarbonsäuren mit sechs Kohlenstoffatomen aus diese mit mindestens Adipinsäuren enthaltenden Gemischen, dadurch gekennzeichnet, daß die Dicarbonsäure(n) mindestens teilweise in die entsprechenden Anhydride umgewandelt wird/werden,
- in Gegenwart eines sauren homogenen Katalysators, d.h. löslich in dem Reaktionsmedium, mit einem pKa-Wert von kleiner oder gleich 5, oder eines sauren heterogenen Katalysators,
- oder in Gegenwart eines Anhydrids oder mehrerer Anhydride in dem Reaktionsmedium, wie Anhydride von aliphatischen Mono- oder Polycarbonsäuren, wobei der Siedepunkt dieser Säuren unter dem der Adipinsäure liegt,
- oder eines Katalysators ausgewählt aus Iridium, Iridiumverbindungen, Rhodium, Rhodiumverbindungen, Palladium, Palladiumverbindungen.

2. Verfahren nach Anspruch 1, dadurch gekennzeichnet, daß die eingesetzten aliphatischen gesättigten Dicarbonsäuren 2-Methylglutarsäure und/oder Ethylbernsteinsäure und/oder Dimethylbernsteinsäure sind.

3. Verfahren nach einem der Ansprüche 1 oder 2, dadurch gekennzeichnet, daß die Anhydridbildung der gesättigten aliphatischen Dicarbonsäuren durch Erhitzen des diese enthaltenden Gemisches auf eine Temperatur von gleich oder großer 60°C durchgeführt wird.

4. Verfahren nach einem der Ansprüche 1 bis 3, dadurch gekennzeichnet, daß es die Destillation der gebildeten Anhydride oder des gebildeten Wassers, entweder im Verlauf der Anhydridbildung oder danach, oder die Abtrennung der Anhydride durch ein anderes Mittel wie Kristallisation umfaßt.

5. Verfahren nach einem der Ansprüche 1 bis 4, dadurch gekennzeichnet, daß es bei einer Temperatur von 80°C bis 350°C durchgeführt wird.

6. Verfahren nach einem der Ansprüche 1 bis 5, dadurch gekennzeichnet, daß es in Gegenwart eines sauren homogenen Katalysators wie Schwefelsäure, Iodwasserstoffsäure, p-Toluolsulfonsäure, Trifluoressigsäure, Trifluormethansulfonsäure, oder eines heterogenen wie Borphosphat, Zirkon oder Sulfonharzen oder auch sauren Tonen, wie insbesondere Smektiten, wie beispielsweise Montmorillonite, Beidellite, Nontronite, Hektorite, Stevensite und Saponite, durchgeführt wird.

7. Verfahren nach einem der Ansprüche 1 bis 6, dadurch gekennzeichnet, daß die Menge des homogenen oder heterogenen sauren Katalysators so ist, daß ein molares Verhältnis Säure/in Anhydrid umzuwandelnde Dicarbonsäure von 0,0005 bis 10, bevorzugt 0,0005 bis 1 oder ein Gewichtsverhältnis von Säure/in Anhydrid umzuwandelnde Dicarbonsäuren von mindestens 5 Prozent vorliegt.

8. Verfahren nach einem der Ansprüche 1 bis 5, dadurch gekennzeichnet, daß es in Gegenwart eines Anhydrids ausgewählt aus Anhydriden von Mono- oder Polycarbonsäuren mit 2 bis 8 Kohlenstoffatomen wie Anhydriden von Essigsäure, Propionsäure, Bernsteinsäure, Valeriansäure, 2-Ethylbernsteinsäure, Dimethylbernsteinsäure, Methylbuttersäure, Methylbutensäure oder Pentensäure wie gegebenenfalls gemischten Anhydriden dieser Säuren durchgeführt wird.

9. Verfahren nach einem der Ansprüche 1 bis 8, dadurch gekennzeichnet, daß die Menge des Anhydrids so ist, daß ein molares Verhältnis Anhydrid/in Anhydrid umzuwandelnde Dicarbonsäure von 0,5 bis 10, bevorzugt 0,5 bis 2 und noch bevorzugter von 1 bis 1,5 vorliegt.

10. Verfahren nach einem der Ansprüche 1 bis 9, dadurch gekennzeichnet, daß die verwendeten Gemische an Dicarbonsäuren Gemische sind, die aus der Wiedergewinnung der Dicarbonsäuren aus Umkristallisationswässern von Adipinsäure oder auch Destillationsrückständen von Dicarbonsäuren stammen, oder aus mehr oder weniger reinen Fraktionen von Arbeitsschritten der Kristallisation, der Raffination oder der Destillation bestehen, die im Rahmen der Herstellung von Adipinsäure durchgeführt wurden.

11. Verfahren nach einem der Ansprüche 1 bis 9, dadurch gekennzeichnet, daß die verwendeten Gemische an Dicarbonsäuren Gemische sind, die aus Verfahren zur Synthese von Adipinsäure hervorgegangen sind.

12. Verfahren nach Anspruch 11, dadurch gekennzeichnet, daß die verwendeten Gemische Gemische von Adipinsäure mit 2-Methylglutarsäure und/oder 2-Ethylbernsteinsäure und/oder Dimethylbernsteinsäure, erhalten während der Hydroxycarbonylierung von Pentensäuren oder der Hydroxycarbonylierung von Butadien oder seiner Derivate sind und gegebenenfalls mehr oder weniger große Mengen des verwendeten Katalysators wie Iridium oder Iridiumverbindungen, Rhodium oder Rhodiumverbindungen, Palladium oder Palladiumverbindungen und/oder der eingesetzten Verbindungen wie Pentensäuren, gegebenenfalls Lösungsmittel, Kokatalysatoren sowie die während der Synthese von Adipinsäure gebildeten Verbindungen wie Valeriansäure, Methylbuttersäuren, Methylbutensäuren, Gamma-Valerolacton enthalten.

13. Verfahren nach einem der Ansprüche 1 bis 12, dadurch gekennzeichnet, daß dem zu behandelnden Gemisch aus Dicarbonsäuren ein Lösungsmittel zugesetzt wird, das eine der in dem Reaktionsgemisch, das aus dem Verfahren der Synthese von Adipinsäure oder der in ein Anhydrid umzuwandelnden Dicarbonsäuren hervorgegangen ist, vorhandenen Verbindungen sein kann, oder von diesen Verbindungen verschieden sein kann.

14. Verfahren nach Anspruch 13, dadurch gekennzeichnet, daß das verwendete Lösungsmittel aus aliphatischen oder cycloaliphatischen Kohlenwasserstoffen, halogenierten, insbesondere chlorierten, aliphatischen oder cycloaliphatischen Kohlenwasserstoffen, aromatischen Kohlenwasserstoffen, aromatischen halogenierten, insbesondere chlorierten, Kohlenwasserstoffen, aliphatischen oder aromatischen oder gemischten Ethern, Carbonsäuren, insbesondere aliphatischen, halogenierten Carbonsäuren, ausgewählt wird.

15. Verfahren nach einem der Ansprüche 13 oder 14, dadurch gekennzeichnet, daß das Lösungsmittel aus Benzol, Toluol, Xylolen, n-Hexan, Dichlormethan, 1,2-Dichlorethan, Cyclohexan, Chlorbenzol, Diphenylether, Dibuthylether, Pentensäuren, Valeriansäure, Gamma-Valerolacton, Essigsäure, Propionsäure, Trifluoressigsäure ausgewählt wird.
